# EUROPÄISCHE PATENTSCHRIFT

(11) **EP 0 967 978 B1**
(45) Veröffentlichungstag und Bekanntmachung des Hinweises auf die Patenterteilung: **07.07.2004**
(21) Anmeldenummer: 98916809.1
(22) Anmeldetag: 24.02.1998
(51) Int. Cl.: A61K 31/425, A61K 31/135

(54) **KOMBINATIONSPRÄPARAT AUS 2-METHYLTHIAZOLIDIN-2,4-DICARBONSÄURE UND PARACETAMOL**
COMBINED PREPARATION OF 2-METHYLTHIAZOLIDINE-2,4-DICARBOXYLIC ACID AND PARACETAMOL
PREPARATION COMBINEE D'ACIDE 2-METHYLTHIAZOLIDINE-2,4-DICARBOXYLIQUE ET DE PARACETAMOL

(30) Priorität: 03.03.1997 DE 19711053
(43) Veröffentlichungstag der Anmeldung: 05.01.2000
(73) Patentinhaber: Susilo, Rudy, Dr., 50999 Köln (DE)
(72) Erfinder: SUSILO, Rudy, D-50999 Köln (DE); WLODEK, Lidia, PL-30-041 Karkow (PL)
(74) Vertreter: Bucher, Ralf, Dipl.-Ing. (Univ.)
(86) Internationale Anmeldenummer: PCT/DE1998/000583
(87) Internationale Veröffentlichungsnummer: WO 1998/038994

(56) Entgegenhaltungen:
- DE-A- 2 116 629
- NAGASAWA ET AL: "2-substituted thiazolidine-4(R)-carboxylic acids as prodrugs of L-cysteine. Protection of mice against acetaminophen hepatotoxicity" J. MED. CHEM., Bd. 27, 1984, Seiten 591-96, XP002075766
- WLODEK ET AL: "formation of 2-Methyl-thiazolidine-2,4-dicarboxylic acid from L-csyteine in rat tissues" ACTA BIOCHIMICA POLONICA, Bd. 31, 1984, Seiten 279-88, XP002075767
- WLODEK ET AL: "2-Methyl-thiazolidine-2,4-dicarboxylic acid protects against paracetamol induced toxicity in human liver derived HepG2 cells" ACTA BIOCHIMICA POLONICA, Bd. 44, Nr. 4, Oktober 1997, Seiten 759-66, XP002075765

## Beschreibung

Die Erfindung betrifft ein Arzneimittel, gekennzeichnet durch einen Gehalt an 2-Methylthiazolidin-2,4-dicarbonsaure und Paracetamol in Kombination.

Langzeitanwendung und/oder hohe Dosen des weitverbreiteten Schmerzmittels Paracetamol (Acetaminophen) führen zum Zelluntergang von Leber- und Nierenzellen. Die Giftigkeit des Paracetamols wird durch einen chemisch reaktiven Metaboliten, das N-Acetyl-p-benzochinonimin (NAPQI) vermittelt. Der Metabolit bindet an Eiweißstoffe der Zelle wie beispielsweise Enzyme, die dadurch inaktiviert werden. Außerdem fuhrt die hohe Dosis von Paracetamol zum Anstieg der Konzentration von Sauerstoffradikalen und aufgrund dessen zum Zusammenbruch des Abwehrsystems der Zelle gegen toxische Einflusse. Durch Messung bestimmter Substanzen im Blutplasma sowie direkt im Gewebe laßt sich sowohl der Grad der Beeintrachtigung der zellulären Abwehrmechanismen als auch der Zerstörung der Zellen beurteilen. Beispiele für solche Indikatorsubstanzen sind die Enzyme Laktatdehydrogenase (LDH), die Transaminasen Alanin-Aminotransferase (AlAT) und Aspartat-Aminotransferase (AspT) im Blutserum sowie Cystein, Cystin, Malondialdehyd, Glutathion, die Konzentration von Sulfhydrylgruppen enthaltenden Proteinen und die Konzentration von kleineren Stoffen, die ebenfalls Sulfhydrylgruppen enthalten.

Das am weitesten verbreitete Antidot gegen eine Paracetamolvergiftung ist N-Acetylcystein. Kombinationspräparate von Paracetamol mit N-Acetylcystein sind jedoch unbekannt. Allgemein wird davon ausgegangen, daß es sich bei N-Acetylcystein um ein Medikament geringer Toxizitat handelt. Allerdings gibt es einige, wenig beachtete Berichte, daß das Toxizitatsrisiko von N-Acetylcystein unterschätzt wird (Estrela, J.M., Saez, G.T., Such, L. und Vina, J., Biochem. Pharmacol. **32:** 3483-3485 (1983) und Vina, J., Romero, F.J., Saez, G.T. und Pallardo, F.V., Experientia **39**: 164-165 (1983)). Wegen des Risikos toxischer Reaktionen von N-Acetylcystein wurde immer wieder nach Alternativen gesucht. Die Applikation von L-Cystein selbst verbietet sich, da die Aminosäure sehr toxisch ist und zum Absterben von Hirnzellen führt (Karlsen, R.L., Grofova, Y., Malthe-Sorensen, D. und Farnum, E., Exp. Brain. Res. **208:** 167-180 (1981)). Eine Möglichkeit, diese Toxizität zu umgehen, ist die Applikation einer sogenannten "prodrug", also eines Vorläufermedikaments, aus der die wirksame Aminosäure erst im Korper kontrolliert freigesetzt wird.

Nagasawa et al in J. Med. Chem (1984, 27, p.591-96) beschreibt die Verwendung von 2-substituierten-Thiazolidin-Derivaten zum Leberschutz beim Einsetzen von Acetaminophen DE2116629 veröffentlicht verschiedene Thiazolidin-Derivate und ihre Verwendung als Arneimittel, zum Beispiel, zum Leberschutz.

Aufgabe der vorliegenden Erfindung ist es, ein Kombinationspräparat zur Verfugung zu stellen, welches die Zelltoxizitat des bewährten Analgetikum/Antipyretikums Paracetamol zu reduzieren vermag.

Die Aufgabe wird erfindungsgemäß dadurch gelost, daß ein Arzneimittel zur Verfügung gestellt wird, welches gekennzeichnet ist durch einen Gehalt an 2-Methylthiazolidin-2,4-dicarbonsäure und Paracetamol in Kombination und pharmazeutisch unbedenklichen Trägerstoffen, Hilfsstoffen und/oder Zusatzstoffen.

Überraschenderweise wurde gefunden, daß 2-Methylthiazolidin-2,4-dicarbonsäure eine Reduktion der Bildung der freien Radikale und eine Erhohung der Sulfhydrylgruppen-Konzentration im Organismus hervorruft. Dies fuhrt zu einer cytoprotektiven und entzündungshemmenden Wirkung dieser Verbindung. Dadurch ist diese Substanz den bisher bekannten Verbindungen, wie N-Acetylcystein deutlich überlegen. Die vom N-Acetylcystein bekannten toxischen Nebenwirkungen können durch die Verwendung von von 2-Methyl-thiazolidin-2,4-dicarbonsäure deutlich verringert werden.

Bei der Freisetzung von L-Cystein aus 2-Methylthiazolidin-2,4-dicarbonsäure entsteht als Nebenprodukt Pyruvat, eine physiologische Substanz, die vollig unschadlich ist. 2-Methyl-thiazolidin-2,4-dicarbonsaure zeichnet sich deshalb im Gegensatz zu N-Acetylcystein durch eine sehr gute Verträglichkeit aus. Es gibt sogar Hinweise auf eine protektive Wirkung von Pyruvat (Rastellini, C., Cicalese, L., Zeevi, A., Mattes, C., Stauko, R.T., Starzl, T.E. und Rao, A.S., Transplant. Proceed. **27:** 3383-3384 (1995)). Pyruvat entsteht physiologischerweise aus Glukose und wird im Zitronensaurezyklus zur Energiegewinnung der Zelle benötigt. Aufgrund dieser Tatsache ist zu erwarten, daß durch eine langsame enzymatische Freisetzung von L-Cystein in den Körperzellen eine retardierende Wirkung erfolgt, die eine im Vergleich mit N-Acetylcystein länger anhaltende Wirkung erwarten läßt.

Überraschenderweise wurde festgestellt, daß nicht nur die Verhinderung der Zerstörung des zellulären Abwehrsystems durch Behandlung mit 2-Methylthiazolidin-2,4-dicarbonsäure und Paracetamol in Kombination bewirkt, sondern auch ein langanhaltender Effekt der Wirkung von 2-Methylthiazolidin-2,4-dicarbonsäure und Paracetamol in Kombination erzielt wird. Dies spricht für eine enzymatisch regulierte Freisetzung von L-Cystein aus 2-Methylthiazolidin-2,4-dicarbonsäure. Außerdem zeigen die Befunde nach einer Monotherapie mit 2-Methylthiazolidin-2,4-dicarbonsäure und Paracetamol in Kombination, daß diese Substanz von der Leber gut vertragen wird.

Die erfindungsgemäßen Arzneimittel können zur oralen, rektalen, subcutanen, intravenösen oder intramuskulären Applikation vorgesehen sein.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zukker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talkum und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talkum, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die folgenden Beispiele erlautern die Erfindung:

### Beispiel 1

### Bestimmung der Zeit-Dosis-Abhängigkeit

### Methoden:

Zunächst wurde ein Zeit- und Dosisabhangigkeit von 2-Methylthiazolidin-2,4-Carbonsäure (CP) und Paracetamol (AA) in Kombination unter in-vivo-Bedingungen bei Mäusen ermittelt und festgestellt, daß bis zu einer Dosis von 2,4 mmol CP/kg Körpergewicht keine toxischen Effekte auftreten. Danach wurden die Mause mit 1,2 mmol CP/kg vorbehandelt und 12 h später mit Kontrollösung bzw. mit 600 mg Paracetamol/kg Körpergewicht i. p. behandelt. Das Lebergewebe und das Blut wurden nach weiteren 3 Stunden gewonnen und die Indikatorsubstanzen gemessen.

### Ergebnisse:

Die Konzentration von Glutathion stieg nach Applikation von 1,2 bzw. 2,4 mmol 2-Methylthiazolidin-2,4-dicarbonsäure/kg Körpergewicht im Lebergewebe nach einer Stunde deutlich an. Dieser Anstieg war noch nach 12 Stunden nachweisbar (Tabelle 1). Dieser langanhaltende Effekt kann damit erklärt werden, daß aus 2-Methylthiazolidin-2,4-dicarbonsäure in der Leberzelle die Aminosäure L-Cystein freigesetzt wird, die der entscheidende Bestandteil des Abwehrsystems der Leberzelle gegen toxische Einflüsse ist. Gluthation enthalt L-Cystein und kann als naturliche Speicherform von L-Cystein aufgefaßt werden.

Eine einzelne Dosis von Paracetamol (600 mg/kg Körpergewicht) führte nach 3 Stunden zu einer enormen Zunahme der aus der Leber freigesetzten Enzyme im Blutserum (AlAT, AspT) als Ausdruck eines Zelluntergangs von Leberzellen. Außerdem nahm die Konzentration der Sauerstoffradikale zu und die Konzentration von Sulfhydrylgruppen, freien Cysteinen und Gykogen drastisch ab (Tabelle 2).

Eine Vorbehandlung mit 2-Methylthiazolidin-2,4-dicarbonsäure (1,2 mmol/kg Körpergewicht) unterdrückte den Anstieg der Leberenzyme im Serum und verminderte die Abnahme der Sulfhydrylgruppen. Cystein und Glykogen sind im Lebergewebe signifikant erhöht. Die Bildung von Sauerstoffradikalen wurde signifikant vermindert.

### Beispiel 2

### In vitro-Untersuchungen an permanenten Leberzellinien

Die Zytotoxizität von Paracetamol und die protektive Wirkung von 2-Methylthiazolidin-2,4-dicarbonsäure wurde an einer permanenten Leberzellinie (Hep G2-Zellen) untersucht. Die Zellen wurden 10 Tage lang in einem Medium gehalten, das Paracetamol (1 mM) enthielt. Eine andere Gruppe von Zellen wurde mit einem Medium, das sowohl Paracetamol als auch 2-Methylthiazolidin-2,4-dicarbonsäure enthielt in gleicher Weise behandelt. Am Ende der Expositionsperiode wurden die Konzentrationen der Indikatorsubstanzen gemessen (Tabelle 3). Die Behandlung der Hep G2-Zellen mit Paracetamol (AA) führte zu einem Anstieg der freien Radikale, der durch gleichzeitige Behandlung mit 2-Methylthiazolidin-2,4-dicarbonsäure (CP) (2 mM) verhindert wurde. Die Konzentrationen von L-Cystein und Cystin sanken leicht nach AA-Exposition. Auch dieser Effekt wurde durch 2-Methylthiazolidin-2,4-dicarbonsaure mehr als kompensiert. Unter den gewählten Bedingungen anderten sich die Konzentrationen der anderen Indikatorsubstanzen nicht. Deshalb sollen in späteren Experimenten höhere Konzentrationen von AA gewählt werden.

Die Befunde mit der Zellkultur bestätigen die protektive Wirksamkeit von 2-Methylthiazolidin-2,4-dicarbonsäure gegenüber hohen Konzentrationen von Paracetamol im Leberzellmodell.

**Tabelle 1**

| Zeit nach Injektion (h) | **GSH µmole/g Feuchtmasse** | | | | |
|---|---|---|---|---|---|
| | Kontrolle 0,9% NaCl x±SD | *CP (1,2 mmol*/*kg) %* | | *CP (2,4 mmol*/*kg) %* | |
| | | x±SD | d. Kontrolle | x±SD | d.Kontrolle |
| 1 | 6,45±0,42 | 7,62^{b}±0,37 | 117,5 | 8,27^{a}±0,38 | 127,6 |
| 4 | 6,38±0,25 | 5,56^{c}±0,42 | 87,2 | ,76±0,42 | 90,3 |
| 8 | 5,97±0,29 | 6,48±0,67 | 108,5 | ,69^{c}±0,32 | 112,0 |
| 12 | 4,73±0,31 | 5,77^{b}±0,30 | 122,0 | ,31^{a}±0,41 | 154,5 |

| | | | | | |
|---|---|---|---|---|---|
| a<0,001 | | | | | |
| b<0,01 | | | | | |
| c<0,02 d<0,05 | | | | | |

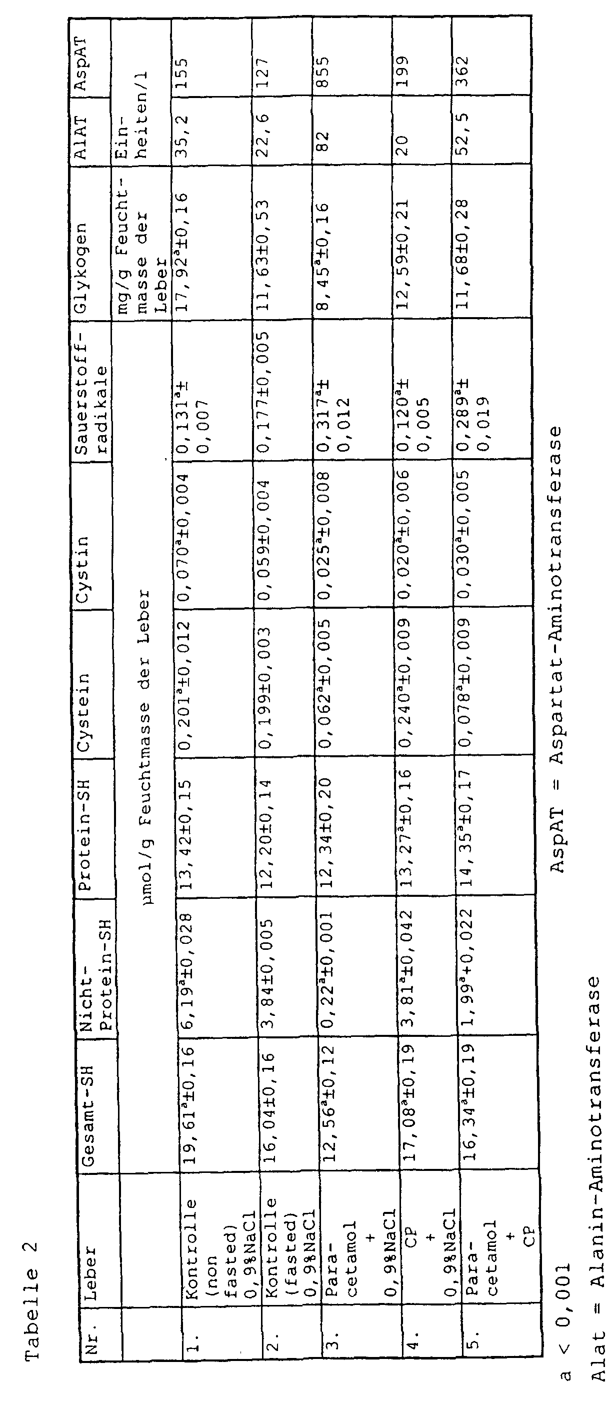

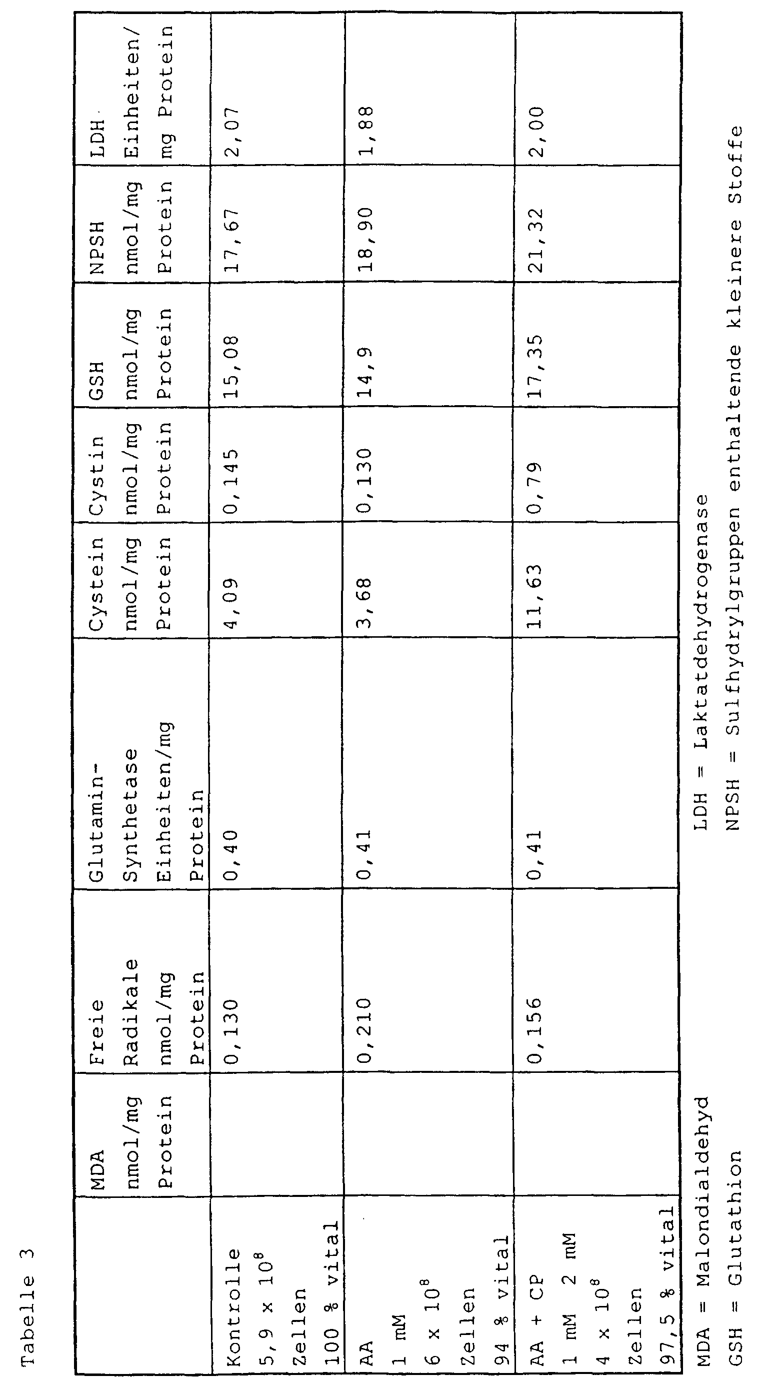

## Patentansprüche

1. Arzneimittel, **gekennzeichnet durch** einen Gehalt an 2-Methylthiazolidin-2,4-dicarbonsäure und Paracetamol in Kombination und pharmazeutisch unbedenklichen Trägerstoffen, Hilfsstoffen und/oder Zusatzstoffen.

2. Verwendung von 2-Methylthiazolidin-2,4-dicarbonsäure und Paracetamol in Kombination als Analgetikum und/oder Antipyretikum.

## Claims

1. Pharmaceutical formulation **characterized by** a content of 2-methylthiazolidine-2,4-dicarboxylic acid in combination with paracetamol and pharmaceutically acceptable carriers, adjuvant, and/or additive.

2. Use of 2-methylthiazolidine-2,4-dicarboxylic acid in combination with paracetamol as analgesic drug and/or antipyretic drug.

## Revendications

1. Médicament, **caractérisé en ce qu'**il contient de l'acide 2-méthylthiazolidin-2,4-dicarboxylique et du paracétamol combinés, et des excipients, adjuvants et / ou additifs neutres d'un point de vue pharmaceutique.

2. Utilisation de l'acide 2-méthylthiazolidin-2,4-dicarboxylique et du paracétamol combinés, en tant qu'analgésique et / ou antipyrétique.
